# EUROPEAN PATENT SPECIFICATION

(11) **EP 3 079 702 B1**
(45) Date of publication and mention of the grant of the patent: **07.03.2018**
(21) Application number: 14792774.3
(22) Date of filing: 21.10.2014
(51) Int. Cl.: A61K 31/55, C08B 37/16, C08L 5/16, A61K 47/69, A61P 9/04

(54) **PHARMACEUTICAL COMPOSITION COMPRISING AMORPHOUS IVABRADINE**
PHARMAZEUTISCHE ZUSAMMENSETZUNG ENTHALTEND AMORPHES IVABRADINE
COMPOSITION PHARMACEUTIQUE COMPRENANT DE L'IVABRADINE AMORPHE

(30) Priority: 12.12.2013 WO PCT/EP2013/076429
(43) Date of publication of application: 19.10.2016
(73) Proprietor: Synthon BV, 6545 CM Nijmegen (NL)
(72) Inventor: EUPEN VAN, Jacobus Theodorus Henricus, NL-6545 CM Nijmegen (NL); KELTJENS, Rolf, NL-6545 CM Nijmegen (NL); MURPANI, Deepak, NL-6545 CM Nijmegen (NL); GARCIA JIMENEZ, Sonia, E-08830 Sant Boi de Llobregat (ES); ÁLVAREZ FERNÁNDEZ, Lisardo, E-08830 Sant Boi de Llobregat (ES)
(86) International application number: PCT/EP2014/072524
(87) International publication number: WO 2015/001133

(56) References cited:
- WO-A1-2014/161131
- WO-A2-2006/013039
- WO-A2-2011/098582
- CN-A- 101 559 228
- CN-A- 103 393 611
- A. Kavanagh: "Developing Amorphous Pharmaceuticals: Opportunity and Necessity", American Pharmaceutical Review - The Review of American Pharmaceutical Business & Technology, 21 August 2012 (2012-08-21), pages 1-7, XP55157921, Retrieved from the Internet: URL:http://www.americanpharmaceuticalrevie w.com/Featured-Articles/119521-Developing- Amorphous-Pharmaceuticals-Opportunity-and- Necessity/ [retrieved on 2014-12-11]

## Description

### BACKGROUND OF THE PRESENT INVENTION

Ivabradine, chemically 3-(3-{[((7*S*)-3,4-dimethoxybicyclo[4.2.0]octa-1,3,5-trien-7-yl)methyl]methylamino}propyl)-7,8-dimethoxy-1,3,4,5-tetrahydro-2*H*-3-benzazepin-2-one of formula (I), 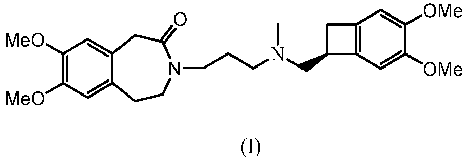 is a pharmaceutically active compound used for the treatment of stable angina pectoris and chronic heart failure. The compound was discovered by Adir and is disclosed in EP534859. The compound may form acid addition salts, for instance ivabradine hydrochloride, which is the active ingredient in the medicinal product sold under the brand name Corlentor® and Procoralan® by Laboratoires Servier.

Ivabradine hydrochloride exhibits polymorphism. WO2006092493 discloses polymorph β of ivabradine hydrochloride, its process of preparation and compositions comprising this polymorph. Polymorph β is the most stable form and is present in the marketed product. Other polymorphic forms of ivabradine hydrochloride are disclosed in WO2005110993, WO2006092491, WO2006092492, WO2006092494, WO2007042656, WO2007042657 and WO2013064307. The prior art thus teaches that ivabradine hydrochloride crystallizes very easily. Moreover, it was experienced in our laboratory that polymorphic transitions of ivabradine hydrochloride take place rather easily, especially in the pharmaceutical compositions.

Amorphous ivabradine hydrochloride and methods for its preparation are disclosed in WO2008146308, CN101597261 and CN101463008. We observed in our laboratory that amorphous ivabradine hydrochloride as such is unstable, very hygroscopic and therefore not suitable for use on pharmaceutical production scale.

WO2011157722 discloses solid molecular dispersions of ivabradine, or a pharmaceutically acceptable salt thereof, with a range of pharmaceutically acceptable excipients. WO2011098582 suggests that amorphous ivabradine hydrochloride could be stabilized by many different stabilizers.

In view of the prior art cited above, there is still a need for pharmaceutical compositions comprising ivabradine, or a pharmaceutically acceptable salt thereof, which are stable, simple to prepare and suitable for use on a commercial scale.

### BRIEF DESCRIPTION OF THE PRESENT INVENTION

The present invention provides a pharmaceutical composition comprising an inclusion complex of ivabradine, or a pharmaceutically acceptable salt thereof, in amorphous form in non-substituted β-cyclodextrin and one or more pharmaceutically acceptable excipients, wherein the weight ratio of ivabradine, or a pharmaceutically acceptable salt thereof, to the non-substituted β-cyclodextrin ranges from 1:1 to 1:2.5. It also provides a process for preparing said inclusion complex by dissolving ivabradine, or a pharmaceutically acceptable salt thereof, and non-substituted β-cyclodextrin in a suitable solvent or solvent mixture, followed by evaporation of the solvent(s). Additionally, the invention provides a process for preparing said pharmaceutical composition comprising mixing or granulating said inclusion complex with one or more excipients, followed by compression into tablets.

Said pharmaceutical composition may be used as a medicament, particularly in the treatment of stable angina pectoris and chronic heart failure.

### DETAILED DESCRIPTION OF THE PRESENT INVENTION

The present invention relates to a pharmaceutical composition comprising an inclusion complex of ivabradine, or a pharmaceutically acceptable salt thereof, in amorphous form in non-substituted β-cyclodextrin and one or more pharmaceutically acceptable excipients, wherein the weight ratio of ivabradine, or a pharmaceutically acceptable salt thereof, to the non-substituted β-cyclodextrin ranges from 1:1 to 1:2.5. Cyclodextrins are compounds made up of sugar molecules bound together in a ring and are composed of 5 or more α-D-glucopyranoside units linked 1→4. Cyclodextrins are produced from starch by means of enzymatic conversion. Three major non-substituted cyclodextrins are known, containing each a different number of glucose monomers ranging from six to eight in a ring, creating a conical shape. The so-called α-cyclodextrin is a six-membered sugar ring molecule, β-cyclodextrin is a seven-membered sugar ring molecule and γ-cyclodextrin is an eight-membered sugar ring molecule.

As the non-substituted cyclodextrins, and in particular β-cyclodextrin, exhibit low aqueous solubility, various semi synthetic derivatives with enhanced aqueous solubility have been developed. One well known example is 2-hydroxypropyl-β-cyclodextrin, which is a partially substituted poly(2-hydroxypropyl)ether of β-cyclodextrin. Its aqueous solubility is high, exceeding 600 mg/ml.

Cyclodextrins may form complexes with various chemicals (guest molecules), in which the chemical is encapsulated inside the cyclodextrin ring and forms a so called inclusion complex. Thereby, original properties of the compound vis-à-vis the cyclodextrin-complexed compound may be modified (Arthur H. Kibbe (Ed.), Handbook of Pharmaceutical Excipients, Third Edition 2000, p. 165-168).

Drugs that can exist in either amorphous or crystalline form tend to crystallize over time when present in amorphous state because the crystalline form of the drug is a lower-energy state than the amorphous form. Active Pharmaceutical Ingredients (APIs) may form inclusion complexes in cyclodextrins wherein the API in the inclusion complex is kept in amorphous form (Thorstein Loftsson at al., Pharmaceutical Sciences, 85(10), 1996, 1017-1025). It is however not self-evident that a given drug will form an inclusion complex with just any cyclodextrin, and that, even in the event the complex is formed, it will be stable over time. Factors playing a role herein are the physicochemical properties of both API and cyclodextrin, the ratio of API to cyclodextrin used and the technique used to prepare the complex. Although cyclodextrin complexation procedures are relatively simple processes, these techniques often require very specific conditions for each guest molecule (John L. Koontz et al., J. Agric. Food Chem., 57(4), 2009, p. 1162-1171).

WO2011098582 suggests that amorphous ivabradine hydrochloride could be stabilized by many different stabilizers, amongst which 2-hydroxypropyl-β-cyclodextrin. Generally, non-substituted β-cyclodextrin, although much cheaper, is less preferred to be used in inclusion complexes because of its low solubility. Surprisingly, it was discovered in our laboratory that non-substituted β-cyclodextrin give nevertheless rise to more stable inclusion complexes with ivabradine, or a pharmaceutically salt thereof, wherein ivabradine, or its salt, is maintained amorphous. During stability studies no conversion into any crystalline form was observed even under stress conditions.

WO2011098582 further states that an excess of 2-hydroxypropyl-β-cyclodextrin, weight ratio 2:9, is employed to ensure the complete complexation of ivabradine in solid form. We were very surprised, when observing the excellent stability of the inclusion complex of the current invention at such weight ratios of ivabradine, or a pharmaceutically acceptable salt thereof, to non-substituted β-cyclodextrin that the ivabradine, or a pharmaceutically acceptable salt thereof, is present in clear excess. In case the weight ratio of ivabradine, or a pharmaceutically acceptable salt thereof, to non-substituted β-cyclodextrin is lowered from 1:2 to 1:1, meaning that the excess of ivabradine, or a pharmaceutically acceptable salt thereof, over non-substituted β-cyclodextrin is further increased, the stability of the pharmaceutical composition is not decreasing. During stability studies no conversion into any crystalline form was observed for both compositions, even under stress conditions. With regard to impurity levels, also no significant differences have been observed between the two compositions; both compositions show sufficient long term stability.

The excellent stability of the inclusion complex of the present invention of ivabradine, or a pharmaceutically acceptable salt thereof, in amorphous form in non-substituted β-cyclodextrin, wherein ivabradine, or a pharmaceutically acceptable salt thereof, is present in excess amount, can not only be explained for by (partial) encapsulation of ivabradine, or a pharmaceutically acceptable salt thereof, in the non-substituted β-cyclodextrin cavity. Ivabradine, or a pharmaceutical acceptable salt thereof, is also accommodated in the intermolecular cavities formed or sandwich-like between layers of non-substituted β-cyclodextrin. As described by József Szejtli (Cyclodextrin Technology, 1988, p. 81), the "intercalation" enhances the molar ratio in favor of the guest molecule, which could account for that, although ivabradine, or a pharmaceutically acceptable salt thereof, is present in excess amounts over the non-substituted β-cyclodextrin, the stability of the pharmaceutical compositions is still excellent. It is however not evident that ivabradine hydrochloride might form stable compositions in the same way with substituted cyclodextrins.

The pharmaceutical compositions prepared with the inclusion complex display dissolution behavior typical for immediate-release formulations. The compositions of the present invention exhibit a dissolution rate of at least 85% in 10 minutes when tested in 500 ml SGF pH 1.2, acetate buffer pH 4.5 or phosphate buffer pH 6.8 in a USP apparatus II at 75 rpm. The pharmaceutical compositions of the present invention behaved similar to Procoralan® in a pilot bioequivalence study.

The weight ratio of ivabradine, or a pharmaceutically acceptable salt thereof, to non-substituted β-cyclodextrin in the inclusion complex ranges from 1:1 to 1:2.5 and is preferably 1:1.

β-Cyclodextrin is the cheapest cyclodextrin, regarded as a safe compound and is widely applied in food and pharmaceutical industry.

At least a major portion of ivabradine, or a pharmaceutically acceptable salt thereof, in the inclusion complex is amorphous. The term "a major portion" of ivabradine, or a pharmaceutically acceptable salt thereof, means that at least 60% of the drug is in amorphous form, rather than a crystalline form. Preferably, ivabradine, or a pharmaceutically acceptable salt thereof, in the inclusion complex is at least 80% in amorphous form. More preferably, ivabradine, or a pharmaceutically acceptable salt thereof, in the inclusion complex is "almost completely amorphous" meaning that the amount of ivabradine, or a pharmaceutically acceptable salt thereof, in the amorphous form is at least 90% as measured by powder X-ray diffraction or any other standard quantitative measurement. Most preferably, ivabradine, or a pharmaceutically acceptable salt thereof, in the inclusion complex is in a completely amorphous form within the detection limits of the techniques used for characterization.

The inclusion complex in accordance with the present invention advantageously is in the form of a free-flowing powder, with excellent handling properties and stable morphology. The inclusion complex is very suitable to be used for the preparation of pharmaceutical compositions.

The pharmaceutical compositions of the present invention comprise the inclusion complex of ivabradine, or a pharmaceutically acceptable salt thereof, in non-substituted β-cyclodextrin and one or more pharmaceutically acceptable excipients. The excipients to be used in accordance with the present invention are well-known and are those excipients which are conventionally used by the person skilled in the art. Depending on the dosage form chosen for the pharmaceutical composition, the person skilled in the art will be able to select suitable pharmaceutically acceptable excipients. Preferably, the dosage form is an immediate release tablet and the pharmaceutically acceptable excipients are chosen from one or more binders, diluents, disintegrants, glidants, lubricants, stabilizers, surface active agents or pH-adjusting agents. More preferably, the composition of the present invention comprises a diluent, a disintegrant and a lubricant.

The diluent to be used in accordance with the present invention may be any diluent known to a person of ordinary skill in the art. Particularly, the diluent to be used in accordance with the present invention is an inorganic diluent, polysaccharide, mono- or disaccharide or sugar alcohol. Microcrystalline cellulose is a particularly preferred diluent.

The disintegrant to be used in accordance with the present invention may be any disintegrant known to a person of ordinary skill in the art. Suitable disintegrants to be used in accordance with the present invention are selected from the group consisting of croscarmellose sodium, crospovidone or sodium starch glycolate. Croscarmellose sodium is a particularly preferred disintegrant.

The lubricant to be used in accordance with the present invention may be any lubricant known to a person of ordinary skill in the art. Magnesium stearate is a particularly preferred lubricant.

In another embodiment of the present invention, at least one of the pharmaceutically acceptable excipients is an intragranular excipient. Preferably, the intragranular excipient to be used in accordance with the present invention is a diluent. Microcrystalline cellulose is a particularly preferred diluent.

In yet another embodiment of the present invention, at least one of the pharmaceutically acceptable excipients is an extragranular excipient. Preferably, the extragranular excipient is chosen from one or more diluents, disintegrants and lubricants. More preferably, the extragranular excipient to be used in accordance with the present invention is a disintegrant. Croscarmellose sodium is a particularly preferred disintegrant. In addition to croscarmellose sodium, the diluent microcrystalline cellulose and the lubricant magnesium stearate may be used as extragranular excipients.

In an advantageous variant of the current invention, the intragranular excipient is microcrystalline cellulose and the extragranular excipients are microcrystalline cellulose, croscarmellose sodium and magnesium stearate.

The pharmaceutical compositions of the present invention display dissolution behavior typical for immediate-release formulations, exhibiting a dissolution rate of at least 85% in 10 minutes when tested in 500 ml SGF pH 1.2, acetate buffer pH 4.5 or phosphate buffer pH 6.8 in a USP apparatus II at 75 rpm. During preparation and storage of the pharmaceutical compositions of the present invention, ivabradine, or a pharmaceutically acceptable salt thereof, remains in the amorphous form.

The present invention further provides a process to prepare an inclusion complex of ivabradine, or a pharmaceutically acceptable salt thereof, in non-substituted β-cyclodextrin, comprising dissolving the cyclodextrin and ivabradine, or a pharmaceutically acceptable salt thereof, in a suitable solvent or solvent mixture, followed by evaporation of the solvent(s), using equipment and methods well-known in the art.

Preferably, the solvent or solvent mixture is water, a polar organic solvent or a mixture of water and a polar organic solvent. Since the non-substituted β-cyclodextrin exhibit a lower solubility compared to the various semi-synthetic derivatives like 2-hydroxypropyl-β-cyclodextrin, the process of the current invention is performed at elevated temperatures in order to dissolve the non-substituted β-cyclodextrin completely. Typically, the mixture of β-cyclodextrin and solvent(s) is heated to temperatures ranging from 50°C to reflux. Preferably, the mixture is heated to 60-70°C.

In an advantageous variant of the process of the present invention, the non-substituted β-cyclodextrin is dissolved in water or a mixture of water and a polar organic solvent by heating and ivabradine, or a pharmaceutically acceptable salt thereof, is added to this solution. Preferred polar organic solvents are alcohols, particularly ethanol or methanol, ethers, particularly tetrahydrofuran, ketones, particularly acetone and acetonitrile. Preferably, water or a mixture of an alcohol and water is used. Advantageously, the inclusion complex is prepared by dissolving non-substituted β-cyclodextrin in water by heating, followed by the addition of ivabradine hydrochloride and subsequent evaporation of the solvent.

The present invention still further provides a process to prepare pharmaceutical compositions comprising an inclusion complex of ivabradine, or a pharmaceutically acceptable salt thereof, in non-substituted β-cyclodextrin and one or more pharmaceutically acceptable excipients. The process comprises mixing or granulating the inclusion complex with one or more pharmaceutically acceptable excipients, followed by compression into tablets, using equipment and methods well-known to the skilled artisan. In an advantageous variant of the process of the present invention, a solution of ivabradine, or a pharmaceutically acceptable salt thereof, and the non-substituted β-cyclodextrin was sprayed over the intragranular excipient(s), e.g. the diluent, in a fluidized bed and the resulting granulate/blend was mixed with one or more pharmaceutically acceptable extragranular excipients, followed by compression into tablets. Preferably, a solution of ivabradine hydrochloride and non-substituted β-cyclodextrin in water was sprayed over microcrystalline cellulose in a fluidized bed, after which the granulate/powder blend was mixed with microcrystalline cellulose, croscarmellose sodium and magnesium stearate, followed by compression into tablets.

The tablets may be optionally further coated by a film-coat. The coating serves generally for cosmetic purposes. The coating material typically has no influence on the release rate, except of an inherent short initial delay in dissolution due to the time necessary to dissolve the coat.

The coating may be selected from amongst one or more of those suitable coating materials known in the art. Coating may be performed by applying one or more film forming polymers, with or without other pharmaceutically inert excipients, as a solution/suspension. Coating is done using any conventional coating technique known in the art, such as spray coating in a conventional coating pan or fluidized bed processor; or dip coating. Preferably, the pharmaceutical composition of the current invention is coated with Opadry® II.

The pharmaceutical compositions of the present invention are packaged in blister pack material. The blister pack materials to be used in accordance with the present invention may be any blister pack material known to a person of ordinary skill in the art. Suitable blister pack materials to be used in accordance with the present invention are selected from the group of PVC/Alu, Duplex/Alu, Triplex/Alu and Alu/Alu. To ensure protection of the compositions of the present invention from *e.g.* moisture and thereby preventing polymorphic conversions, Triplex/Alu and Alu/Alu are particularly preferred blister pack materials. After storage of the pharmaceutical compositions in these blister pack materials for 6 months at 40°C/75% RH, XRPD analysis showed no reflections in accordance with crystalline ivabradine hydrochloride.

The pharmaceutical composition in accordance with the present invention may be used as a medicament. The pharmaceutical composition typically may be used in the treatment of stable angina pectoris and chronic heart failure.

The following examples are intended to illustrate the scope of the present invention.

### EXAMPLES

### Example 1, ivabradine hydrochloride:β-cyclodextrin (weight ratio 1:2)

80 ml of water was added to 8.0 g of β-cyclodextrin. The suspension was heated until a clear solution was obtained. 4.0 g of ivabradine hydrochloride was added and the clear solution was allowed to cool to ambient temperature. The mixture was concentrated *in vacuo.* The residue was dried over night at 40°C under vacuum.

The XRPD pattern of the isolated inclusion complex does not show any reflections in accordance with crystalline ivabradine hydrochloride. XRPD analysis performed 4 weeks after storing the powder in an alu bag at 55°C/90% RH and 6 months at 40°C/75% RH showed still no reflections in accordance with crystalline ivabradine hydrochloride.

### Example 2, ivabradine hydrochloride:β-cyclodextrin (weight ratio 1:1)

Tablets comprising an inclusion complex of ivabradine hydrochloride and β-cyclodextrin with a weight ratio of 1:1 with a composition as presented in the table were prepared using the process as described below:

| **Ingredients** | **Quantity (mg/tablet)** |
|---|---|
| **Intragranular phase** | |
| Ivabradine hydrochloride | 8.1 (equals 7.5 mg ivabradine free base) |
| β-Cyclodextrin | 8.1 |
| Microcrystalline cellulose | 161.5 |
| Purified water | q.s. |

| Extragranular phase | |
|---|---|
| Microcrystalline cellulose | 53.9 |
| Croscarmellose sodium | 7.2 |
| Magnesium stearate | 1.2 |
| **Tablet weight - uncoated** | **240.0** |
| Opadry® II 32F | 7.2 |
| **Tablet weight - coated** | **247**.**2** |

### Manufacturing process:

Water was heated to 60-70°C and β-cyclodextrin was dissolved under stirring. Ivabradine hydrochloride was added and the mixture was stirred until complete dissolution was achieved. The solution was kept at 60-70°C. The intragranular microcrystalline cellulose fraction was sieved using a suitable mesh sieve for deagglomeration and it was added into the fluid bed. The solution containing ivabradine hydrochloride and β-cyclodextrin was sprayed over the microcrystalline cellulose in the fluid bed under heating (about 60°C) and stirring. The resulting granulate/powder blend is dried to appropriate Loss on Drying value. The granulate/powder blend is sieved through an appropriate mesh size sieve. The rest of the microcrystalline cellulose and the croscarmellose sodium were sieved through a suitable mesh size sieve for deagglomeration. The excipients were mixed with the sieved granulate/powder blend in a suitable blender. The magnesium stearate was sieved through an appropriate sieve to deagglomerate and mixed with the powder mix in a suitable blender. The homogeneous powder obtained, was compressed using a rotary tabletting machine using appropriate punches. The tablets were coated with a water suspension of Opadry® II 32F until 3% weight gain. The tablets were packed in suitable packaging material.

XRPD analysis performed after storing the tablets in several types of packaging material (Triplex 120/Alu, Alu/Alu) for 6 months at 40°C/75% RH showed no reflections in accordance with crystalline ivabradine hydrochloride.

The tablets obtained, exhibited a dissolution rate of at least 85% in 10 minutes when tested in 500 ml SGF pH 1.2, acetate buffer pH 4.5 or phosphate buffer pH 6.8 in a USP apparatus II at 75 rpm. The dissolution profiles of the tablets were similar to the profiles of Procoralan®.

The tablets behaved similar to Procoralan® in a pilot bioequivalence study.

### Example 3, ivabradine hydrochloride:β-cyclodextrin (weight ratio 1:2)

Tablets comprising an inclusion complex of ivabradine hydrochloride and β-cyclodextrin with a weight ratio of 1:2 with a composition as presented in the table were prepared using the process as described below:

| **Ingredients** | **Quantity (mg/tablet)** |
|---|---|
| **Intragranular phase** | |
| Ivabradine hydrochloride | 8.1 (equals 7.5 mg ivabradine free base) |
| β-Cyclodextrin | 16.2 |
| Microcrystalline cellulose | 155.5 |
| Purified water | q.s. |

| **Extragranular phase** | |
|---|---|
| Microcrystalline cellulose | 51.8 |
| Croscarmellose sodium | 7.2 |
| Magnesium stearate | 1.2 |
| **Tablet weight - uncoated** | **240.0** |
| Opadry® II 32F | 7.2 |
| **Tablet weight** - **coated** | **247**.**2** |

### Manufacturing process:

Water was heated to 60-70°C and β-cyclodextrin was dissolved under stirring. Ivabradine hydrochloride was added and the mixture was stirred until complete dissolution was achieved. The solution was kept at 60-70°C. The intragranular microcrystalline cellulose fraction was sieved using a suitable mesh sieve for deagglomeration and it was added into the fluid bed. The solution containing ivabradine hydrochloride and β-cyclodextrin was sprayed over the microcrystalline cellulose in the fluid bed under heating (about 60°C) and stirring. The resulting granulate/powder blend is dried to appropriate Loss on Drying value. The granulate/powder blend is sieved through an appropriate mesh size sieve. The rest of the microcrystalline cellulose and the croscarmellose sodium were sieved through a suitable mesh size sieve for deagglomeration. The excipients were mixed with the sieved granulate/powder blend in a suitable blender. The magnesium stearate was sieved through an appropriate sieve to deagglomerate and mixed with the powder mix in a suitable blender. The homogeneous powder obtained, was compressed using a rotary tabletting machine using appropriate punches. The tablets were coated with a water suspension of Opadry® II 32F until 3% weight gain. The tablets were packed in suitable packaging material.

XRPD analysis performed after storing the tablets in several types of packaging material (Triplex 120/Alu, Alu/Alu) for 6 months at 40°C/75% RH showed no reflections in accordance with crystalline ivabradine hydrochloride.

The tablets obtained exhibited a dissolution rate of at least 85% in 10 minutes when tested in 500 ml SGF pH 1.2, acetate buffer pH 4.5 or phosphate buffer pH 6.8 in a USP apparatus II at 75 rpm. The dissolution profiles of the tablets were similar to the profiles of Procoralan®.

The tablets behaved similar to Procoralan® in a pilot bioequivalence study.

## Claims

1. A pharmaceutical composition comprising an inclusion complex of ivabradine, or a pharmaceutically acceptable salt thereof, in amorphous form in non-substituted β-cyclodextrin and one or more pharmaceutically acceptable excipients, wherein the weight ratio of ivabradine, or a pharmaceutically acceptable salt thereof, to the non-substituted β-cyclodextrin ranges from 1:1 to 1:2.5.

2. The composition according to claim 1 comprising the hydrochloric acid salt of ivabradine.

3. The composition according to claim 1 or 2, wherein the pharmaceutical composition is a tablet and the pharmaceutically acceptable excipients are one or more binders, diluents, disintegrants, glidants, lubricants, stabilizers, surface active agents or pH-adjusting agents.

4. The composition according to any one of claims 1 to 3, wherein at least one of the pharmaceutically acceptable excipients is an intragranular excipient.

5. The composition according to claim 4, wherein the intragranular excipient is a diluent.

6. The composition according to any one of claims 1 to 5, wherein at least one of the pharmaceutically acceptable excipients is an extragranular excipient.

7. The composition according to claim 6, wherein the extragranular excipient is a disintegrant.

8. The composition according to any one of claims 1 to 7 exhibiting a dissolution rate of at least 85% in 10 minutes when tested in 500 ml SGF pH 1.2, acetate buffer pH 4.5 or phosphate buffer pH 6.8 in a USP apparatus II at 75 rpm.

9. The composition according to any one of claims 1 to 8 packed in Triplex/Alu or Alu/Alu blister pack material.

10. A process for preparing the inclusion complex according to claim 1 or 2 comprising dissolving ivabradine, or a pharmaceutically acceptable salt thereof, and non-substituted β-cyclodextrin in a suitable solvent or solvent mixture, followed by evaporation of the solvent(s).

11. The process according to claim 10, wherein the solvent is a polar solvent selected from the group consisting of alcohols, ethers, ketones, acetonitrile and water or a mixture thereof.

12. The process according to claim 10 or 11 further comprising mixing or granulating the inclusion complex with one or more pharmaceutically acceptable excipients, followed by compression into tablets.

13. The composition according to any one of claims 1 to 9 for use in the treatment of stable angina pectoris and chronic heart failure.

## Patentansprüche

1. Pharmazeutische Zusammensetzung, die einen Inklusionskomplex von Ivabradin, oder einem pharmazeutisch verträglichen Salz davon, in amorpher Form in nicht substituiertem β-Cyclodextrin und einen oder mehrere pharmazeutisch verträgliche Hilfsstoffe umfasst, wobei das Gewichtsverhältnis von Ivabradin, oder einem pharmazeutisch verträglichen Salz davon, zum nicht substituierten β-Cyclodextrin im Bereich von 1:1 bis 1:2,5 liegt.

2. Zusammensetzung nach Anspruch 1, die das Salzsäure-Salz von Ivabradin umfasst.

3. Zusammensetzung nach Anspruch 1 oder 2, wobei die pharmazeutische Zusammensetzung eine Tablette ist und die pharmazeutisch verträglichen Hilfsstoffe ein oder mehrere Bindemittel, Verdünnungsmittel, Sprengmittel, Gleitmittel, Schmiermittel, Stabilisatoren, oberflächenaktive Mittel oder pHeinstellende Mittel sind.

4. Zusammensetzung nach einem beliebigen der Ansprüche 1 bis 3, wobei mindestens einer der pharmazeutisch verträglichen Hilfsstoffe ein intragranulärer Hilfsstoff ist.

5. Zusammensetzung nach Anspruch 4, wobei der intragranuläre Hilfsstoff ein Verdünnungsmittel ist.

6. Zusammensetzung nach einem beliebigen der Ansprüche 1 bis 5, wobei mindestens einer der pharmazeutisch verträglichen Hilfsstoffe ein extragranulärer Hilfsstoff ist.

7. Zusammensetzung nach Anspruch 6, wobei der extragranuläre Hilfsstoff ein Sprengmittel ist.

8. Zusammensetzung nach einem beliebigen der Ansprüche 1 bis 7, die eine Auflösungsgeschwindigkeit von mindestens 85% in 10 Minuten aufweist, wenn sie in 500 ml SGF pH 1,2, Acetatpuffer pH 4,5 oder Phosphatpuffer pH 6,8 in einem USP-Apparat II bei 75 upm getestet wird.

9. Zusammensetzung nach einem beliebigen der Ansprüche 1 bis 8, die in Triplex/Alu- oder Alu/Alu-Blisterverpackungsmaterial verpackt ist.

10. Verfahren zum Herstellen des Inklusionskomplexes gemäß Anspruch 1 oder 2, das Auflösen von Ivabradin, oder einem pharmazeutisch verträglichen Salz davon, und nicht substituiertem β-Cyclodextrin in einem geeigneten Lösungsmittel oder Lösungsmittelgemisch, gefolgt von Evaporation des/der Lösungsmittel(s) umfasst.

11. Verfahren nach Anspruch 10, wobei das Lösungsmittel ein polares Lösungsmittel ist, das aus der Gruppe bestehend aus Alkoholen, Ethern, Ketonen, Acetonitril und Wasser oder einem Gemisch davon ausgewählt ist.

12. Verfahren nach Anspruch 10 oder 11, das weiterhin Mischen oder Granulieren des Inklusionskomplexes mit einem oder mehreren pharmazeutisch verträglichen Hilfsstoffen, gefolgt von Verpressung zu Tabletten umfasst.

13. Zusammensetzung nach einem beliebigen der Ansprüche 1 bis 9 zur Verwendung in der Behandlung von stabiler Angina Pectoris und chronischer Herzinsuffizienz.

## Revendications

1. Une composition pharmaceutique comprenant un complexe d'inclusion d'ivabradine, ou d'un de ses sels pharmaceutiquement acceptables, sous forme amorphe dans de la β-cyclodextrine non substituée et un ou plusieurs excipients pharmaceutiquement acceptables, dans laquelle le rapport pondéral de l'ivabradine, ou d'un de ses sels pharmaceutiquement acceptables, à la β-cyclodextrine non substituée est compris entre 1/1 et 1/ 2,5.

2. La composition selon la revendication 1, comprenant le sel d'acide chlorhydrique de l'ivabradine.

3. La composition selon la revendication 1 ou 2, dans laquelle la composition pharmaceutique est un comprimé et les excipients pharmaceutiquement acceptables sont un ou plusieurs agents liants, diluants, désintégrants, glissants, lubrifiants, stabilisants, tensioactifs ou d'ajustement du pH.

4. La composition selon l'une quelconque des revendications 1 à 3, dans laquelle au moins l'un des excipients pharmaceutiquement acceptables est un excipient intragranulaire.

5. La composition selon la revendication 4, dans laquelle l'excipient intragranulaire est un diluant.

6. La composition selon l'une quelconque des revendications 1 à 5, dans laquelle au moins l'un des excipients pharmaceutiquement acceptables est un excipient extragranulaire.

7. La composition selon la revendication 6, dans laquelle l'excipient extragranulaire est un désintégrant.

8. La composition selon l'une quelconque des revendications 1 à 7 présentant un taux de dissolution d'au moins 85% en 10 minutes lors d'essais dans 500 ml de SGF pH 1,2, tampon acétate de pH 4,5 ou tampon phosphate de pH 6,8 dans un appareil USP II à 75 tr/min.

9. La composition selon l'une quelconque des revendications 1 à 8 conditionnée dans un matériau pour blister formé de Triplex/Alu ou Alu/Alu.

10. Un procédé de préparation du complexe d'inclusion selon l'une des revendications 1 ou 2, comprenant la dissolution de l'ivabradine ou d'un de ses sels pharmaceutiquement acceptables et de la β-cyclodextrine non substituée dans un solvant ou mélange de solvants approprié, suivie de l'évaporation du/des solvant (s).

11. Le procédé selon la revendication 10, dans lequel le solvant est un solvant polaire choisi dans le groupe constitué par les alcools, les éthers, les cétones, l'acétonitrile et l'eau ou un mélange de ceux-ci.

12. Le procédé selon la revendication 10 ou 11, comprenant en outre le mélange ou la granulation du complexe d'inclusion avec un ou plusieurs excipients pharmaceutiquement acceptables, suivi de la compression en comprimés.

13. La composition selon l'une quelconque des revendications 1 à 9, pour une utilisation dans le traitement de l'angine de poitrine stable et de l'insuffisance cardiaque chronique.
